# EUROPEAN PATENT APPLICATION

(11) **EP 4 621 057 A1**
(43) Date of publication of application: **24.09.2025**
(21) Application number: 23892085.4
(22) Date of filing: 17.11.2023
(51) Int. Cl.: C12N 15/63, C12Q 1/6897, C12Q 1/06, C12N 9/52, G01N 33/52

(54) **METHOD FOR DETECTION OR ACTIVITY MEASUREMENT OF CELL-BASED BOTULINUM TOXIN BY USING RECOMBINANT FLUORESCENT PROTEIN**

(30) Priority: 18.11.2022 KR 20220155414; 13.11.2023 KR 20230156468
(71) Applicant: Biolinks Inc., Cheonan-si, Chungcheongnam-do 31156 (KR)
(72) Inventor: KIM, Jin Yeop, Hwaseong-si, Gyeonggi-do 18476 (KR)
(74) Representative: M. Zardi & Co S.A.
(86) International application number: PCT/KR2023/018595
(87) International publication number: WO 2024/107023

(57) **Abstract**

The present invention relates to a method for detecting or determining botulinum toxin activity using a cell-based assay, and more particularly, to a recombinant cell comprising a gene construct including a recombinant fluorescent protein coding sequence, a botulinum toxin recognition sequence and a peptide coding sequence for inhibiting fluorescent protein expression, and to the use thereof. According to the method of the present invention, botulinum toxin can be detected or its activity quantitatively analyzed by fluorescence measurement without the need for using cell lines that are difficult to culture, such as stem cells.

## Description

### [TECHNICAL FIELD]

The present invention relates to a cell-based assay method for detecting or determining botulinum toxin activity, and more particularly, to a recombinant cell introduced with a gene construct comprising a recombinant fluorescent protein coding sequence, a botulinum toxin recognition sequence and a peptide coding sequence for inhibiting expression of the fluorescent protein, and the use thereof.

### [BACKGROUND OF THE INVENTION]

The generally accepted method for detecting botulinum toxin (BoNT) present in food, clinical, or environmental samples is a mouse LD₅₀ bioassay (mLD₅₀). In particular, in the pharmaceutical field, mLD₅₀ is utilized as the standard assay method for determining the biological activity of botulinum toxin used for aesthetic or medical purposes. However, the mouse LD₅₀ bioassay for verifying botulinum toxin activity is significantly influenced by the examiner, test institution and facilities, making it difficult to accurately and reproducibly quantify the biological potency of botulinum toxin.

Therefore, in order to minimize the number of animals used in experiments and the animals' pain, and to standardize the method for determining botulinum toxin activity-that is, the potency assay of botulinum toxin-as well as to encourage the development of alternative methods to mLD₅₀, a ZEBET meeting was held (Adler et al., ATLA Alternatives to Laboratory Animals, Vol. 38(4), pp. 315-330, 2010). In various countries, many research institutes and industrial entities have initiated studies to develop cell-based potency assays (CBPAs) or cell-based bioassays (CBBs) that are satisfactory alternatives to mLD₅₀ in terms of specificity, sensitivity and reproducibility. For successful implementation of CBPAs or CBBs, it is required to (1) obtain monoclonal or polyclonal antibodies specific to SNAP25₁₉₇, and (2) acquire cells that exhibit high sensitivity to botulinum toxin at low concentrations (~pM). In the early 2004s, Dr. Chapman and his colleagues invented a fluorescence reporter assay utilizing botulinum toxin fused with two fluorescent proteins (Dong et al., PNAS, Vol. 101(41), pp. 14701-14706, 2004), and later commercialized it under the name BoCell^{™} assay (BioSentinel Inc).

As described above, global efforts to develop CBPAs as alternatives to the mLD₅₀ assay are ongoing, and ultimately, animal-based analytical methods are expected to be removed. The development of effective CBPAs will facilitate the broader application of various botulinum toxin products, enhance quality control standards, and promote greater consumer confidence, thereby strengthening the competitiveness of botulinum toxin products.

Allergan, the manufacturer of BOTOX^{®}, successfully established a monoclonal antibody specific to SNAP25₁₉₇ and SiMa cells which us an ideal host cell line highly sensitive to botulinum toxin-human neuroblastoma (Fernandez-Salas et al., PLoS ONE, 7(11): e49516, 2012). In 2010, Allergan developed a new CBPA using the aforementioned antibody and cell line, and it was approved by the FDA as the first CBPA capable of replacing the mLD₅₀ assay (US8455213B2 and US2010/0204559A1). Another botulinum toxin manufacturer, Merz (Germany), developed a distinct CBA-ELISA in 2014, which employs a unique in-situ fixation method for neuronal cells and is characterized by its ability to penetrate the cell membrane and immunologically detect endogenous SNAP25.

The CBPA developed by Allergan and the CBA-ELISA developed by Merz demonstrate excellent sensitivity, equivalent to mouse-based assay methods, even at EC₅₀ levels below picomolar concentrations (i.e., <1.0 U/well). The technical platforms of both Allergan and Merz utilize general commercial rabbit polyclonal antibodies (e.g., Sigma S9684) to detect SNAP25 under optimized conditions. In terms of cell lines, Allergan's CBPA exclusively employs differentiated human neuroblastoma, SiMa cells, whereas Merz's CBA-ELISA uses differentiated human induced pluripotent stem cells (iPS cells) as a standardized and optimized host cell.

However, these previously developed CBPAs determine the endogenous levels of SNAP25₁₉₇ and SNAP25_{FL} using Western blotting, and thus are not well-suited for high-throughput assay (HTA) applications.

Under this technical background, the present inventors endeavored to develop a cell-based assay system capable of determining botulinum toxin activity regardless of the type of cell line. As a result, the inventors completed the present invention by confirming that, upon treatment of cells introduced with a gene construct comprising a recombinant fluorescent protein, a botulinum toxin recognition sequence and a peptide for inhibiting expression of the fluorescent protein, with botulinum toxin, the activity could be detected below picomolar concentrations comparable to existing analytical methods, and the activity could also be quantitatively measured.

### [DETAILED DESCRIPTION OF THE INVENTION]

### [PROBLEMS TO BE SOLVED]

One of the purposes of the present invention is to provide a recombinant cell for detecting or determining botulinum toxin activity using a peptide for inhibiting expression of the fluorescent protein.

Another purpose of the present invention is to provide a method for determining botulinum toxin activity using the above recombinant cell.

Still another purpose of the present invention is to provide a method for detecting or determining botulinum toxin activity, and a method for evaluating botulinum toxin inhibitors, using the above recombinant cell.

Another purpose of the present invention is to provide a kit for detecting or determining botulinum toxin activity.

### [SOLUTION]

The present invention will now be described in further detail as follows. It should be noted, however, that the respective descriptions and embodiments disclosed herein may be applied to other descriptions and embodiments as well. That is, all combinations of the various elements disclosed in the present invention fall within the scope of the invention.

Furthermore, the scope of the present invention should not be considered as being limited by the following detailed descriptions.

One aspect of the present invention relates to a recombinant cell for detecting or determining botulinum toxin activity, into which a gene construct comprising a recombinant fluorescent protein coding sequence, a botulinum toxin recognition sequence, and a peptide coding sequence for inhibiting expression of the fluorescent protein has been introduced.

The term "botulinum toxin" as used in the present invention refers to a neurotoxic protein produced by the bacterium *Clostridium botulinum.* The genus *Clostridium* comprises more than 127 species, which are distinguished by their morphology and function. *Clostridium botulinum* is an anaerobic, Gram-positive bacterium that produces botulinum toxin, a potent polypeptide neurotoxin that causes the paralytic disease botulism in humans and animals. Spores of C. *botulinum* are found in soil and can grow in improperly sterilized and sealed food containers, which are common sources of botulism. Symptoms of botulism typically appear 18 to 36 hours after ingestion of food contaminated with C. *botulinum* cultures or spores. The toxin appears capable of crossing the intestinal epithelium without a significant loss of toxicity and exhibits high affinity for cholinergic motor neurons. Botulinum toxin intoxication can result in symptoms such as impaired locomotion, dysphagia, dysarthria, paralysis of the respiratory muscles, and ultimately death. Botulinum toxin is clinically used for treating neuromuscular disorders characterized by hyperactive skeletal muscles, i.e., movement disorders.

In 1989, botulinum toxin type A complex was approved by the U.S. Food and Drug Administration (FDA) for the treatment of essential blepharospasm, strabismus, and hemifacial spasm. Subsequently, botulinum toxin type A also received FDA approval for the treatment of cervical dystonia and glabellar lines (frown lines), while botulinum toxin type B was approved for the treatment of cervical dystonia. Compared to botulinum toxin type A, other serotypes of botulinum toxin are generally believed to have lower potency and/or shorter duration of action. The clinical effect of intramuscular injection of botulinum toxin type A typically appears within one week after administration. The duration of symptomatic relief from a single intramuscular injection of botulinum toxin type A is on average approximately three months, although significantly longer therapeutic effects have also been reported. Although all botulinum toxin serotypes are believed to inhibit the release of the neurotransmitter acetylcholine at the neuromuscular junction, they act on different neurosecretory proteins and cleave these proteins at different sites. For example, botulinum toxin types A and E both cleave the 25-kDa synaptosomal-associated protein (SNAP25), but they target different amino acid sequences within the protein.

Botulinum toxin types B, D, F and G act on vesicle-associated membrane protein (VAMP, also known as synaptobrevin), and each serotype cleaves a different site on the protein.

Finally, botulinum toxin type C1 appears to cleave both syntaxin and SNAP25. These differences in mechanisms of action are likely to affect the relative potency and/or duration of activity of the various botulinum toxin serotypes. Particularly, substrates of botulinum toxin can be found in a variety of different cell types. *In vitro* studies showed that botulinum toxin inhibited potassium ion-induced release of both acetylcholine and norepinephrine from primary cell cultures of brainstem tissue. In addition, botulinum toxin inhibited the evoked release of glycine and glutamate from primary cultures of spinal cord neurons, and it has also been reported to inhibit the release of several neurotransmitters, including acetylcholine, dopamine, norepinephrine, CGRP, Substance P, and glutamate, from brain synaptosome preparations. Accordingly, when used at an appropriate concentration, botulinum toxin suppresses stimulus-evoked release of most neurotransmitters.

In the present invention, the term "toxin activity" refers to the intrinsic toxicity or potency of botulinum toxin, which is defined based on the standard assay method, the mouse LD₅₀ bioassay (mLD₅₀). Specifically, 1 unit (1U) of botulinum toxin is defined as the amount that results in a 50% lethality rate in mice weighing approximately 20 grams. Botulinum toxin, particularly botulinum toxin serotype A, is commercially available in a freeze-dried or vacuum-dried form. However, during the dilution process performed immediately prior to clinical application or due to the storage time of the diluted solution, the potency of the botulinum toxin may be reduced. In such cases, the expected therapeutic effect of the botulinum toxin may not be achieved. Therefore, it is essential to accurately predict and determine the potency of botulinum toxin.

In the present invention, the recombinant cell can be a prokaryotic cell, an animal cell or a plant cell.

The prokaryotic cell mentioned herein can be selected from the group consisting of *Escherichia coli, Rhizobium, Bifidobacterium, Rhodococcus, Candida, Erwinia, Enterobacter, Pasteurella, Mannheimia, Actinobacillus, Aggregatibacter, Xanthomonas, Vibrio, Pseudomonas, Azotobacter, Acinetobacter, Ralstonia, Agrobacterium, Rhizobium, Rhodobacter, Zymomonas, Bacillus, Staphylococcus, Lactococcus, Streptococcus, Lactobacillus, Clostridium, Corynebacterium, Streptomyces, Bifidobacterium,* and *Cyclobacterium,* but is not limited thereto.

In the present invention, the animal cells can be derived from primary neurons, cultured neurons (established neurons and human neurons), non-neuronal cells, neuroblastoma, spinal cord neurons, dorsal root ganglion neurons, cerebral cortex neurons, cerebellar neurons, hippocampal neurons, or motor neurons. More preferably, the cells may be selected from the group consisting of SiMa, N2042F clonal cell lines, SH-SY5Y cell lines, Neuro2A cell lines, N1E-115 cell lines, NG108-15 cell lines, SH-N-SH cell lines, and general animal cancer cell lines used for experimental research, such as HeLa, HEK293, A549, U2OS can be selected as the cells but are not limited thereto.

In the present invention, the recombinant fluorescent protein can be any protein that exhibits fluorescence through intracellular protein expression, without limitations. Any recombinant fluorescent protein that can emit light without affecting fluorescence expression may be used. Preferably, the fluorescent proteins may be selected from the group consisting of green fluorescent protein (GFP), blue fluorescent protein (BFP), cyan fluorescent protein (CFP), yellow fluorescent protein (YFP), red fluorescent protein (RFP) and orange fluorescent protein (OFP), or recombinantly produced from those, but are not limited thereto.

In the present invention, the GFP may be AGFP (480, 505nm), AzamiGreen (492, 505nm), EGFP (484, 507nm), Emeraid (487, 509nm), GFP (475, 509nm), GFP_uv (395, 509nm), GFP_S65T (488, 510nm), mWasabi (493, 509nm), Stemmer (395, 509nm), sfGFP (485, 510nm), TagGFP (482, 505nm), T-Sapphire (399, 511nm), TurboGFP (482, 502nm), or ZsGreen (493, 505nm), but is not limited thereto, and the nm values for each GFP represent the absorption and emission wavelengths, respectively.

In the present invention, the BFP may be Azurite (384, 459nm), EBFP (383, 445nm), EBFP2 (383, 448nm), mTagBFP (399, 456nm) or Y66H (382, 459nm), but is not limited thereto, and the nm values for each BFP represent the absorption and emission wavelengths, respectively.

In the present invention, the CFP may be ECFP (439, 476nm), AmCyan1 (458, 489nm), Cerulean (433, 475nm), CyPet (435, 477nm), mECFP (433, 475nm), MidoriishiCyan (472, 495nm), mTEP1 (462, 492nm), or TagCFP (458, 480nm), but is not limited thereto, and the nm values for each CFP represent the absorption and emission wavelengths, respectively.

In the present invention, the YFP may be EYFP (514, 527nm), mBanana (540, 553nm), mCitrine (516, 529nm), PhiYFP (525, 537nm), TagYFP (508, 524nm), Topaz (514, 527nm), Venus (515, 528nm), YPet (517, 530nm), or ZsYellow1 (529, 539nm), but is not limited thereto, and the nm values for each YFP represent the absorption and emission wavelengths, respectively.

In the present invention, the RFP may be AQ143 (595, 655nm), AsRed2 (576, 592nm), dKeima-Tandem (440, 620nm), HcRed1 (588, 618nm), tHcRed (590, 637nm), JRed (584, 610nm), mApple (568, 592nm), mCherry (587, 610nm), mPlum (590, 637nm), mRaspberry (598, 625nm), mRFP1 (584, 607nm), mRuby (558, 605nm), or mStrawberry (574, 596nm), but is not limited thereto, and the nm values for each RFP represent the absorption and emission wavelengths, respectively.

In the present invention, the OFP may be DsRed/RFP (558, 583nm), DsRed2 (563, 582nm), DsRed-Express (555, 584nm), DsRed-Monomer (556, 586nm), Tomato (554, 581nm), tdTomato (554, 581nm), Kusabira orange (548, 559nm), mKO2 (551, 565nm), mOrange (548, 562nm), mOrange2 (549, 565nm), mTangerine (568, 585nm), TagRFP (555, 584nm) or TagREP-T (555, 584nm), but is not limited thereto, and the nm values for each OFP represent the absorption and emission wavelengths, respectively.

In the present invention, the botulinum toxin recognition sequence refers to a sequence encoding a peptide that acts as a substrate for botulinum toxin and is cleaved or degraded by the botulinum toxin. Preferably, the botulinum toxin recognition sequence comprises the amino acid at position 197 of the amino acid sequence represented by SEQ ID NO. 1 and includes 6 to 56 adjacent amino acids, and, in the case of the N-terminus, may include up to amino acid 202, but is not limited thereto.

In the present invention, the botulinum toxin recognition sequence can further comprise amino acids and adjacent amino acids at positions other than the amino acid at position 197 of the amino acid sequence represented by SEQ ID NO. 1 and the adjacent amino acids.

The term "adjacent amino acids" may refer to, when including 6 amino acids adjacent to the 197th position of the amino acid sequence represented by SEQ ID NO. 1, 1 amino acid on the N-terminal side and 5 amino acids on the C-terminal side; 2 amino acids on the N-terminal side and 4 on the C-terminal side; or 3 on both sides. For example, in the case of including 20 adjacent amino acids centered around the 197th position of the amino acid sequence represented by SEQ ID NO. 1, the sequence may include 1 amino acid on the N-terminal side and 19 amino acids on the C-terminal side, 2 amino acid on the N-terminal side and 18 amino acids on the C-terminal side, 3 amino acid on the N-terminal side and 17 amino acids on the C-terminal side, 4 amino acid on the N-terminal side and 16 amino acids on the C-terminal side, or 5 amino acid on the N-terminal side and 15 amino acids on the C-terminal side.

SEQ ID No. 1 (SNAP25 full sequence):

MAEDADMRNELEEMQRRADQLADESLESTRRMLQLVEESKDAGIRTLVMLDE

QGEQLDRVEEGMNHINQDMKEAEKNLKDLGKCCGLFICPCNKLKSSDAYKKA

WGNNQDGVVASQPARVVDEREQMAISGGFIRRVTNDARENEMDENLEQVSGII

GNLRHMALDMGNEIDTQNRQIDRIMEKADSNKTRIDEANQRATKMLGSG

The botulinum toxin recognition sequence of the present invention may include a nucleic acid sequence encoding a SNARE protein, and specifically, may be a nucleic acid sequence encoding a portion of the SNAP25 peptide, and more specifically, may be the nucleic acid sequence represented by SEQ ID NO. 9, but not limited thereto.

In the present invention, the peptide for inhibiting fluorescence expression may be any peptide capable of quenching the recombinant fluorescent protein of the present invention without limitation. Preferably, the peptide may be transmembrane domain (TMD) of influenza M2 protein or may be characterized by being structurally α-helical and capable of inducing the binding of identical domains, but is not limited thereto. Specifically, the peptide for inhibiting fluorescence expression in the present invention may be a quencher peptide, and more specifically, may be a peptide expressed by the nucleic acid sequence represented by SEQ ID NO. 10, but is not limited thereto.

In the present invention, the gene construct may be introduced using a cell-penetrating peptide, a lipid-based gene carrier, or a combination thereof, or via methods such as electroporation or magnetofection, and known methods in the art may also be utilized.

The gene construct of the present invention may be characterized by including the nucleic acid sequences represented by SEQ ID NOs. 8 to 10, but is not limited thereto.

The gene construct of the present invention may be included in a vector. As used herein, the term "vector" refers to a gene construct containing a gene sequence operably linked to suitable regulatory sequences capable of expressing the gene in an appropriate host. The vector may be a plasmid, a phage particle, or simply a potential genomic insert. When transformed into a suitable host, the vector can replicate and function independently of the host genome, or in some cases, may be integrated into the genome itself. Since plasmids are currently the most commonly used form of vectors, the terms "plasmid" and "vector" are sometimes used interchangeably in the specification. However, the present invention includes other forms of vectors that are known or will become known in the art and that have equivalent functions.

In order to express the gene sequence of the present invention, any of a wide variety of expression regulatory sequences may be used in the vector. Examples of useful expression regulatory sequences include the early and late promoters of SV40 or adenovirus, the *lac* system, the *trp* system, the *tac* or *trc* systems, T3 and T7 promoters, the major operator and promoter regions of phage lambda, the regulatory regions of fd code protein, promoters for 3-phosphoglycerate kinase or other glycolytic enzymes, promoters for the above-mentioned phosphatases such as *Pho5,* promoters of yeast alpha-mating system, and other constitutive or inducible sequences known to regulate the expression of genes in prokaryotic or eukaryotic cells or in their viruses, as well as various combinations thereof. The T7 RNA polymerase promoter Φ may be usefully employed for protein expression in *E. coli.*

In the present invention, the term "expression vector" generally refers to a recombinant carrier into which a fragment of a heterologous gene is inserted, typically a doublestranded gene fragment. Here, a heterologous gene refers to a foreign gene that is not naturally found in a host cell. Once inside the host cell, the expression vector can replicate independently of the host chromosomal genes, allowing multiple copies of the vector and its inserted (heterologous) gene to be produced.

As is well known in the art, in order to enhance the expression level of the transformed gene in a host cell, the gene must be operably linked to transcriptional and translational regulatory sequences that are functional in the selected host. Preferably, the expression regulatory sequences and the gene are included within a single expression vector that also contains a bacterial selection marker and an origin of replication. If the expression host is a eukaryotic cell, the expression vector must also include a useful expression marker suitable for use in eukaryotic expression systems.

A host cell that is transformed or transfected with the above-described expression vector constitutes another aspect of the present invention.

As used herein, the term "transformation" refers to the introduction of a gene into a host such that the gene can be replicated either as an extrachromosomal element or by integration into the chromosome. The term "transfection" refers to the acceptance of an expression vector by a host cell, regardless of whether the coding sequence is actually expressed.

The host cell of the present invention may be a prokaryotic or eukaryotic cell. Typically, host cells with high gene uptake efficiency and high expression efficiency of the introduced gene are used. Examples of such host cells include well-known prokaryotic and eukaryotic cells such as *E. coli, Pseudomonas, Bacillus, Streptomyces,* fungi, and yeast.

Of course, it would be understood that not all vectors and expression regulatory sequences function equally well in expressing the genetic sequence of the present invention. Likewise, not all hosts perform equally well, nor do all hosts function the same in a given expression system. However, one skilled in the art can select appropriate vectors, expression regulatory sequences, and hosts without undue experimental burden and without departing from the scope of the present invention. For example, in selecting a vector, the host must be taken into consideration, as the vector must be able to replicate within the host. Considerations must also include the copy number of the vector, the ability to control that copy number, and the expression of other proteins encoded by the vector, such as antibiotic resistance markers. Similarly, in selecting expression regulatory sequences, several factors must be considered. These include, for example, the relative strength of the sequence, its inducibility, and compatibility with the gene sequence of the present invention-particularly in terms of potential secondary structures. Unicellular hosts should be selected by taking into account factors such as the selected vector, the toxicity of the product encoded by the gene sequence of the present invention, its secretion properties, the host's ability to properly fold the protein, culture and fermentation requirements, and the ease of purification of the gene product from the host.

In the present invention, botulinum toxin is a neurotoxic protein produced by *Clostridium botulinum,* and the botulinum toxin can be classified into seven serotypes: A, B, C (C1, C2), D, E, F, and G. Each of the serotypes cleaves different sites of different neurosecretory proteins. Specifically, botulinum toxin serotypes A and E cleave SNAP25 (Synaptosomal nerve-associated protein 25), while serotypes B, D, F, and G cleave VAMP (Vesicle-associated membrane protein). Serotype C1 cleaves both syntaxin and SNAP25, thereby exerting its neurotoxic effects.

In the present invention, the botulinum toxin recognition sequences for each serotype are as shown in Table 1 below.

**[Table 1]**

| TYPE | SEQ ID No. | botulinum toxin recognition sequences |
|---|---|---|
| A | 2 | (SNAP25 protein) IMEKADSNKTRIDEANQ/RATKMLGSG |
| B | 3 | (VAMP protein) LSELDDRADALQAGASQ/FETSAAKL |
| C | 2 | (SNAP25 protein) IMEKADSNKTRIDEANQR/ATKMLGSG |
| | 4 | (Syntaxin protein) EHAVDYVERAVSDTKK/AVKYQSKARRKKIM |
| D | 5 | (VAMP protein) |
| | | VNVDKVLERDQK/LSELDDRADALQAGASQFETSAAKL |
| E | 6 | HMALDMGNEIDTQNRQIDR/IMEKADSNKTRIDEANQRATKML GSG (SNAP25 protein) |
| F | 5 | (VAMP protein) |
| | | VNVDKVLERDQ/KLSELDDRADALQAGASQFETSAAKL |
| G | 7 | (VAMP protein) |
| | | DKVLERDQKLSELDDRADALQAGASQFESSA/AKLKRKYWW |

In the amino acid sequences of Table 1, the symbol "/" indicates the cleavage site acted upon by each serotype of botulinum toxin.

In the present invention, the botulinum toxin is preferably botulinum toxin serotype A, B, or E, and more preferably botulinum toxin serotype A, but is not limited thereto.

That is, the recombinant cell into which the gene construct of the present invention has been introduced can express a fusion protein in which the recombinant fluorescent protein, the botulinum toxin recognition peptide, and the peptide for inhibiting fluorescence expression are sequentially linked. The expressed fusion protein does not exhibit fluorescence or color in the absence of botulinum toxin, but upon contact with botulinum toxin, the botulinum toxin recognition peptide is cleaved, resulting in the separation of the fluorescent protein from the peptide for inhibiting fluorescence expression, thereby producing fluorescence or color.

Another aspect of the present invention relates to a cell-based method for detecting or determining botulinum toxin activity, comprising: culturing recombinant cells of the present invention; treating the cultured recombinant cells with botulinum toxin; and measuring fluorescence intensity or the number of cells exhibiting fluorescence in the botulinum toxin-treated recombinant cells.

A further aspect of the present invention relates to a cell-based method for evaluating a botulinum toxin inhibitor, comprising: culturing recombinant cells of the present invention; treating the cultured recombinant cells with botulinum toxin and a candidate inhibitor; and determining the activity of the botulinum toxin by measuring fluorescence intensity or the number of cells exhibiting fluorescence and comparing the results with a control group.

In the present invention, the culturing recombinant cells can be carried out using commonly used culture media. For example, any medium typically used for animal cell culture may be employed, including but not limited to Eagle's MEM (Eagle, H. Science 130:432 (1959)), α-MEM (Stanner, C.P. et al., Nat. New Biol. 230:52 (1971)), Iscove's MEM (Iscove, N. et al., J Exp. Med. 147:923 (1978)), Medium 199 (Morgan et al., Proc Soc. Exp Bio Med. 73:1 (1950)), CMRL 1066, RPMI 1640 (Moore et al., J. Amer. Med. Assoc. 199:519 (1950)), F12 (Ham, Proc. Natl. Acad. Sci. USA 53:288 (1965)), F10 (Ham, R.G. Exp. Cell Res. 29:515 (1963)), DMEM (Dulbecco's Modified Eagle's Medium, Dulbecco, R. et al., Virology 8:396 (1959)), a mixture of DMEM and F12 (Barnes, D. et al., Anal. Biochem. 102:255 (1980)), Waymouth's MB752/1 (Waymouth, C. J. Natl. Cancer Inst. 22:1003 (1959)), McCoy's 5A (McCoy, T.A., et al., Proc. Soc. Exp. Biol. Med. 100:115 (1959)), and the MCDB series (Ham, R.G. et al., In Vitro 14:11 (1978)). Detailed information regarding such media can be found in R. Ian Freshney's Culture of Animal Cells: A Manual of Basic Technique, Alan R. Liss, Inc., New York, which is incorporated herein by reference.

Another aspect of the present invention relates to a kit for detecting or determining botulinum toxin activity, comprising a probe that includes a fluorescent substance, a botulinum toxin-recognition peptide, and a fluorescence expression inhibitor.

In the present invention, the fluorescent substance may be selected from the group consisting of FAM (6-carboxyfluorescein), HEX, Texas Red, JOE, CY5, CY3 and Alexa680, but is not limited thereto.

In the present invention, the botulinum toxin-recognition peptide refers to a peptide expressed by the "botulinum toxin recognition sequence" described above.

In the present invention, the fluorescence expression inhibitor may be TAMRA (6-carboxytetramethyl-rhodamine), BHQ1, BHQ2, or Dabcyl, but is not limited thereto.

In the present invention, the probe may be a protein or chemical substance that directly contacts the sample specimen, but is not limited thereto.

**In** the present invention, the kit may be a form of a bottle, tub, sachet, envelope, tube, ampoule or the like, and these may be partially or wholly formed from materials such as plastic, glass, paper, foil or wax. The container may be equipped with a cap that is fully or partially detachable, and may initially be part of the container or attached thereto by mechanical, adhesive, or other means. The container may also be fitted with a stopper that can be pierced by a needle to access the contents. The kit may include an external package, which may further include instructions for use describing how to utilize the components of the kit.

**In** the present invention, the kit may be characterized in that, when botulinum toxin is present in a sample specimen, the botulinum toxin recognition peptide is cleaved by the botulinum toxin, resulting in the emission of fluorescence from the previously quenched fluorescent substance.

**In** the present invention, the term "sample specimen" encompasses a variety of samples, and may specifically include those collected from one or more of liquids, soil, air, food, waste, the gastrointestinal tracts of animals or plants, and animal or plant tissues, but is not limited thereto. Specifically, the liquids may include water, blood, urine, tears, sweat, saliva, lymph, and cerebrospinal fluid, among others. The water may include river water, seawater, lake water, and rainwater. Waste may include sewage and wastewater. The term "animal or plant" also includes the human body. Furthermore, the animal or plant tissues may include tissues such as mucosa, skin, epidermis, hair, scales, eyes, tongue, cheeks, hooves, beaks, snouts, feet, hands, mouth, nipples, ears, and nose.

**In** the case of analyzing a sample derived from a mammal or of human origin, the sample may be obtained from specific tissues or organs. Representative examples of tissues include connective tissue, skin, muscle, or nerve tissue. Representative examples of organs include the eyes, brain, lungs, liver, spleen, bone marrow, thymus, heart, lymph, blood, bones, cartilage, pancreas, kidneys, gallbladder, stomach, small intestine, testis, ovary, uterus, rectum, nervous system, glands, and internal blood vessels. The biological samples to be analyzed may include any cells, tissues, fluids or other media derived from biological sources that can be effectively analyzed using the present invention. This also includes samples derived from food prepared for human or animal consumption.

In addition, the biological samples to be analyzed include body fluid samples, which may comprise blood, plasma, lymph, breast milk, urine, feces, ocular fluid, saliva, semen, brain extracts (e.g., brain homogenates), cerebrospinal fluid, and extracts of tissues such as the appendix, spleen and tonsils, but are not limited thereto.

### [BENEFITS OF THE INVENTION]

The present invention relates to a cell-based assay system for determining botulinum toxin activity, and a method for detecting and determining botulinum toxin activity using the system. The system enables the detection or quantitative analysis of botulinum toxin activity through fluorescence measurement without the need to use cell lines which it is difficult to culture such as stem cells.

### [BREIF DESCRIPTION OF DRAWINGS]

FIG. 1 shows a vector containing the gene construct of the present invention and the results of gene sequence analysis and quantitative analysis using the vector.
FIG. 2 depicts the result of comparing the fluorescence expression levels between cells introduced with the gene construct of the present invention and control cells introduced with a conventional fluorescent expression construct.
FIG. 3 illustrates the results of confirming botulinum toxin activity by treating cells containing the gene construct of the present invention with botulinum toxin.
FIG. 4 shows the results of evaluating botulinum toxin activity in cells introduced with the gene construct of the present invention depending on the concentration of fetal bovine serum (FBS) in the culture medium.
FIG. 5 illustrates the analysis process for evaluating the efficacy of botulinum toxin using cells introduced with the gene construct of the present invention.
FIG. 6 depicts the results of the efficacy analysis of botulinum toxin using cells introduced with the gene construct of the present invention.

### [DETAILED DESCRIPTION OF THE INVENTION]

In the present invention, it was investigated whether the botulinum toxin activity could be quantitatively measured using a cell line expressing a recombinant fusion protein which is designed to enable emission of fluorescence when the fluorescence of a quenched recombinant fluorescent protein is restored upon cleavage by botulinum toxin.

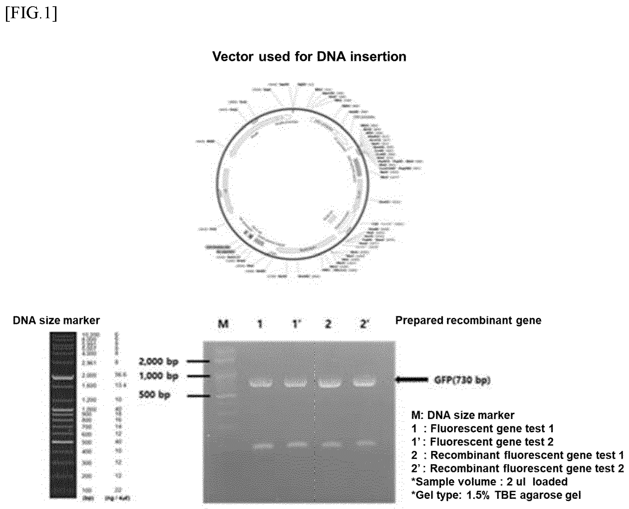

Specifically, in one embodiment of the present invention, a gene construct encoding a recombinant fluorescent protein amino acid sequence-SNAP25 protein botulinum toxin A cleavage site- peptide sequence for inhibiting fluorescence expression was prepared in linear form as a vector suitable for cellular introduction, followed by ligation to recombine the construct into a circular form. Thereafter, quantitative gene analysis was performed to confirm whether the recombinant gene construct was successfully ligated, and the result was compared with that of a general fluorescent protein-expressing vector (see FIG. 1).

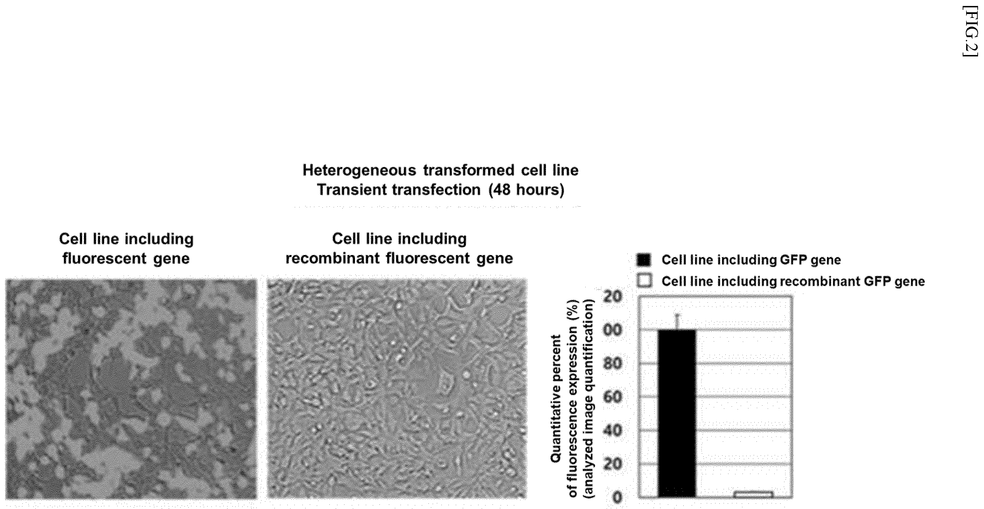

In another embodiment of the present invention, a gene construct encoding a general fluorescent protein expression sequence or a gene construct encoding a recombinant fluorescent protein amino acid sequence-SNAP25 protein botulinum toxin A cleavage site-peptide sequence for inhibiting fluorescence expression was introduced into cells by heterogeneous transformation. As a result of the fluorescence expression, it was revealed that cells introduced with the general fluorescent protein expression construct exhibited fluorescence, whereas cells introduced with the recombinant construct showed suppressed fluorescence expression (see FIG. 2).

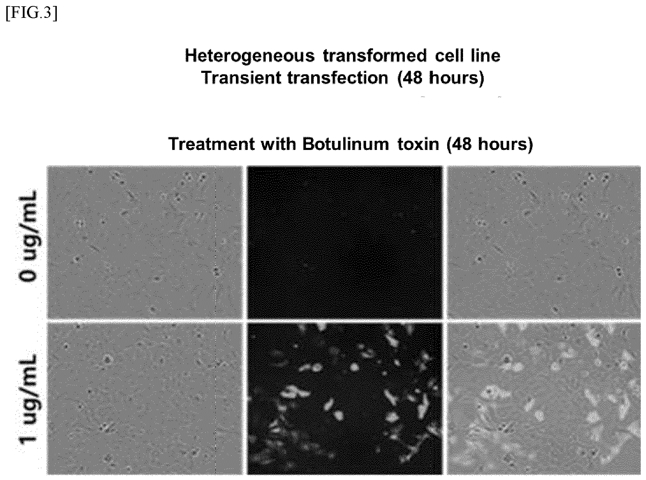

In another embodiment of the present invention, the recombinant gene construct encoding the fluorescent protein amino acid-SNAP25 protein botulinum toxin A cleavage site-peptide sequence for inhibiting fluorescence expression was heterogeneously introduced into cells and thereby the cell was transformed. Thereafter, it was confirmed that the fluorescence expression inhibited by botulinum toxin A was restored when botulinum toxin A was treated (see FIG. 3).

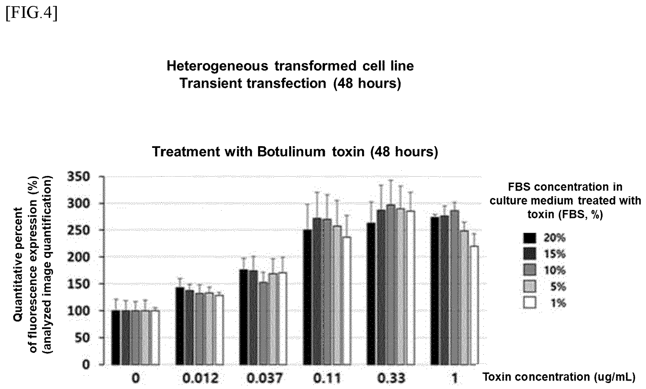

In another embodiment of the present invention, a gene construct encoding a recombinant fluorescent protein amino acid sequence-SNAP25 protein botulinum toxin A cleavage site-peptide sequence for inhibiting fluorescence expression was introduced into cells via heterogeneous transformation. Upon treatment with botulinum toxin, it was confirmed that fluorescence expression was induced in proportion to the activity of the botulinum toxin. Furthermore, it was confirmed that the level of fluorescence expression remained proportional to the botulinum toxin activity regardless of the serum concentration in the toxin-treated medium (see FIG. 4).

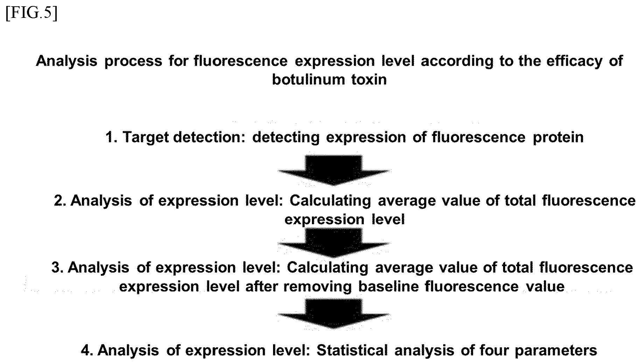

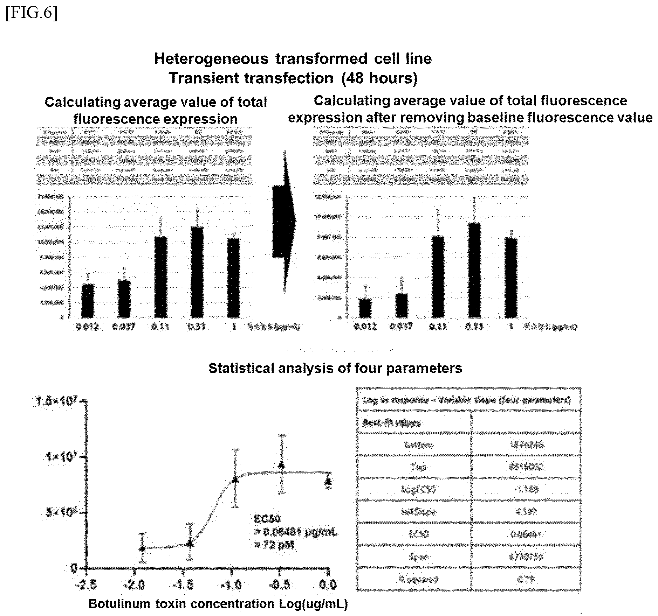

In another embodiment of the present invention, an analysis process for evaluating the efficacy of botulinum toxin was established using cells introduced with a gene construct encoding a recombinant fluorescent protein amino acid sequence-SNAP25 protein botulinum toxin A cleavage site-peptide sequence for inhibiting fluorescence expression (see FIG. 5). The results of fluorescence expression analysis conducted according to the process are shown in FIG. 6.

Hereinafter, the present invention will be described in further detail with reference to the following examples. The examples are provided solely for illustrative purposes and are not intended to limit the scope of the present invention. It will be apparent to those skilled in the art that various modifications and variations can be made without departing from the spirit or scope of the invention.

### EXAMPLE 1. Construction of gene construct

A plasmid of a gene construct comprising the sequence of Table 2 below was constructed.

**[Table 2]**

| SEQ ID No. | Name | Sequence |
|---|---|---|
| 8 | EGFP | ATGGTGAGCAAGGGCGAGGAGCTGTTCACCGGGGTGGT GCCCATCCTGGTCGAGCTGGACGGCGACGTAAACGGCC ACAAGTTCAGCGTGTCCGGCGAGGGCGAGGGCGATGCC ACCTACGGCAAGCTGACCCTGAAGTTCATCTGCACCAC CGGCAAGCTGCCCGTGCCCTGGCCCACCCTCGTGACCA CCCTGACCTACGGCGTGCAGTGCTTCAGCCGCTACCCC GACCACATGAAGCAGCACGACTTCTTCAAGTCCGCCAT GCCCGAAGGCTACGTCCAGGAGCGCACCATCTTCTTCA AGGACGACGGCAACTACAAGACCCGCGCCGAGGTGAA GTTCGAGGGCGACACCCTGGTGAACCGCATCGAGCTGA AGGGCATCGACTTCAAGGAGGACGGCAACATCCTGGG GCACAAGCTGGAGTACAACTACAACAGCCACAACGTCT ATATCATGGCCGACAAGCAGAAGAACGGCATCAAGGT GAACTTCAAGATCCGCCACAACATCGAGGACGGCAGCG TGCAGCTCGCCGACCACTACCAGCAGAACACCCCCATC GGCGACGGCCCCGTGCTGCTGCCCGACAACCACTACCT GAGCACCCAGTCCGCCCTGAGCAAAGACCCCAACGAG AAGCGCGATCACATGGTCCTGCTGGAGTTCGTGACCGC CGCCGGGATCACTCTCGGCATGGACGAGCTGTACAAG |
| 9 | BoNT/A | GATGAGGCCAACCAACGTGCAACAAAGATG |
| 10 | Quencher | TGCAATGATTCGTCAGACCCTCTGGTCGTGGCTGCCTCC ATTATTGGTATTCTGCACCTGATCCTGTGGATTCTGGAT CGTCTG |

After synthesizing the gene in the order of recombinant fluorescent protein amino acid - botulinum toxin A cleavage sequence of SNAP25 protein -peptide sequence for inhibiting fluorescent protein expression, sub-cloning was performed using a multicloning site (MCS). Subsequently, gene sequence analysis and quantitative gene analysis were conducted using the completed vector, confirming the insertion of the desired sequence (see FIG. 1).

The detailed method is as follows:
Preparation of Lysogeny Broth (LB) Medium for Bacterial Culture (per 1 liter): 25 grams of LB medium powder was added to 1 liter of distilled water in a 2-liter glass bottle. A magnetic stir bar was inserted into the bottle and the solution was stirred using a magnetic stirrer until completely dissolved. The stir bar was removed and the bottle was loosely capped. The solution was sterilized in an autoclave at 121 °C for 15 minutes. After sterilization, the medium was made cool, and aliquoted as needed, and stored in a refrigerator. (Before use, the medium was placed at room temperature.)

Preparation of LB Agar Plates for Bacterial Culture (per 1 liter): 25 grams of LB medium powder and 12 grams of agar were added to 1 liter of distilled water in a 2-liter glass bottle. The bottle was closed with a cap and sterilized in an autoclave at 121 °C for 15 minutes. 25 grams of LB medium powder and 12 grams of agar were added to 1 liter of distilled water in a 2-liter glass bottle. A magnetic stir bar was inserted to the bottle and the solution was stirred using a magnetic stirrer until completely dissolved. The stir bar was removed and the bottle was loosely capped. The solution was sterilized in an autoclave at 121 °C for 15 minutes. Once the sterilized medium has cooled to a warm temperature, 1 mL of ampicillin solution (concentration: 100 mg/mL) was added to the sterilized medium and the solution was mixed well. 20 mL of the ampicillin-supplemented medium was poured into each sterile petri dish and the agar was allowed to be solidified at room temperature. After complete solidification, the LB agar plates were inverted and stayed dry in a 37 °C incubator for 8-10 hours. The plates were sealed with parafilm and stored in a refrigerator. (Before use, the plates were placed in a 37 °C incubator for at least 30 minutes.)

Preparation of 0.1 M Calcium Chloride Solution: 1.11 grams of calcium chloride was dissolved in 100 mL of distilled water in a 200 mL glass bottle. The bottle was loosely capped and the solution was sterilized in an autoclave at 121 °C for 15 minutes. After sterilization, the solution was cooled, the cap was securely closed, and the solution was stored in a refrigerator.

Preparation of 50% Glycerol Solution: 25 mL of glycerol and 25 mL of distilled water were mixed in a 100 mL glass bottle. The bottle was loosely capped, and the mixture was sterilized in an autoclave at 121 °C for 15 minutes. After sterilization, the solution was allowed to cool, the cap was securely closed, and the solution was stored in a refrigerator.

Preparation of Competent Cell Stock Solution: Competent cells were taken from -80°C storage and thawed on ice for 5-10 minutes. Subsequently, 50-100 µL of the thawed cells were added to 10 mL of lysogeny broth (LB) medium without antibiotics in a 50 mL conical tube and gently mixed. The tube was loosely capped and incubated in a shaking incubator at 37°C for over 16 hours. After 16 hours, once the culture appeared turbid, 1 mL of the culture was inoculated into 100 mL of pre-sterilized LB medium in a 250 mL Erlenmeyer flask, followed by incubation at 37°C in a shaking incubator for 2 hours. Subsequently, 100 µL of the culture was taken to measure the bacterial cell concentration (OD600). The optical density (OD) was measured at 600 nm, and a range of 0.4-0.6 in OD value was desirable. When OD value was below 0.4, incubation was continued in 30-60 minute intervals until the OD value reached a range of 0.4-0.6. When the OD value reached the target range, the culture was placed on ice for 10 minutes. It was then divided into 50 mL aliquots into pre-chilled, sterilized 5 mL conical tubes, and centrifuged at 5,000 rpm for 10 minutes. After centrifugation, the supernatant was discarded, and 20 mL of cold 0.1 M calcium chloride was added to the cell pellet. The cells were gently resuspended and left on ice for 15 minutes. The suspension was then aliquoted and centrifuged at 5,000 rpm for 10 minutes, and the supernatant was removed. Next, 4 mL of cold 0.1 M calcium chloride was added to the cell pellet, and the bacterial cells were gently resuspended and then incubated on ice for 30 minutes. During the incubation, sterilized microtubes were pre-filled with 100 µL of cold 50% glycerol and kept on ice(the process was performed under as cold conditions as possible). After 30-minute incubation, 4 mL of the resuspended bacterial cells were mixed with the pre-aliquoted 100 µL of 50% glycerol in the sterilized microtubes. The mixture was divided into 100 µL aliquots, capped, and rapidly frozen in liquid nitrogen. Once all bacterial cells were aliquoted, the liquid nitrogen was removed and the frozen competent cell stock solutions were stored at -80°C until use(for future use, the competent cells were thawed on ice prior to application).

Preparation of 1 M Tris aminomethane (pH 7.5): 12.11 g of Tris aminomethane was dissolved in 70 mL of distilled water in a beaker. The pH was adjusted to 7.5 using hydrochloric acid, and the final volume was brought up to 100 mL with distilled water. The solution was filtered and kept refrigerated.

Preparation of 1 M Magnesium Chloride: 2.03 g of magnesium chloride was dissolved in 10 mL of distilled water in a 50 mL conical tube. The solution was then filtered and kept refrigerated.

Preparation of 0.5 M Dithiothreitol: 0.7712 g of dithiothreitol was dissolved in 10 mL of distilled water in a 50 mL conical tube. The solution was filtered and kept refrigerated.

Preparation of 5× T5 exonuclease-dependent mixed buffer (1 mL): In a 1.5 mL conical tube, 500 µL of 1 M Tris aminomethane (pH 7.5), 50 µL of 1 M magnesium chloride and 100 µL of 0.5 M dithiothreitol were combined. Then, 0.25 g of polyethylene glycol 8000 was added and completely dissolved by heating. After complete dissolution, the solution was placed on ice, and 350 µL of distilled water was added to adjust the final volume to 1 mL. Subsequently, 1 µL of T5 exonuclease (final concentration 10 U/mL) was added, and the mixture was gently mixed. The prepared buffer was aliquoted into 100 µL portions and stored at -80°C until use (thawed on ice before use).

Preparation of PCR Products: Primers (forward and reverse) were prepared as concentrated stock solutions at a concentration of 100 pmole/µL by dissolving them in distilled water. 100 pmole/µL of primer stock was then respectively diluted 10-fold with distilled water to obtain a concentration of 10 pmole/µL. The template DNA was also diluted with distilled water to a concentration of 1 ng/µL and 5 µL of the solution was prepared. In a microtube containing 20 µL of PCR reaction mixture, 13 µL of distilled water was added. Subsequently, 1 µL each of the forward and reverse primers at a concentration of 10 pmole/µL was added to the reaction mixture. Then, 5 µL of the template DNA at a concentration of 1 ng/µL was added. The reaction mixture was gently tapped with a finger and centrifuged using a mini centrifuge to collect the contents at the bottom of the tube and placed into PCR machine. The final reaction volume was adjusted to 20 µL, and the PCR was performed according to the cycling conditions described in Table 3.

**[Table 3]**

| Step | Temperature | Reaction time | The number of cycle |
|---|---|---|---|
| Pre-denaturation | 95°C | 5 min | 1 |
| Denaturation | 95°C | 30 sec | 30~35 |
| Primer annealing | 55°C | 30 sec | |
| Extension | 72°C | 1 min | |
| | | for sequences with fewer than 1000 base pairs | |
| Final extension | 72°C | 5 min | 1 |
| Hold | 4°C | Unlimited | unlimited |

Preparation of linear form of the pcDNA 3.1 vector: 700 to 800 nanograms of the circular pcDNA 3.1 vector was placed into a sterilized PCR tube. The reaction mixture was prepared according to the components described in Table 4.

**[Table 4]**

| Sample | Volume (µl) |
|---|---|
| pcDNA 3.1 vector (1000 ng) | 0.8 |
| 10x concentrated Quickcut Buffer | 3 |
| Nhe I (restriction enzyme) | 1 |
| Kpn I (restriction enzyme) | 1 |
| Distilled water | 24.2 |
| Final volume | 30 |

A final volume of 30 µL of the sample was prepared and mixed thoroughly by pipetting. The solution was then centrifuged to pellet. The reaction was made at 37°C for 15 minutes. After the 15-minute reaction, the cleaved vector was purified using the Doktoprep Gel/Polymerase Chain Reaction Purification Kit. The purification was carried out in the following sequence.

30 µL of the cleaved vector was added to a sterilized microtube, and 150 µL of the polymerase chain reaction (PCR) binding solution was added. The mixture is gently mixed by tapping with a finger, followed by centrifugation to pellet(the ratio of cleaved vector sample to PCR binding solution is 1:5). 180 µL of the mixture was added to a spin column, and centrifugation was performed at 13,000 rpm for 1 minute at room temperature. The eluate collected in the recovery tube of the spin column was discarded, and the spin column was reinserted into the recovery tube. 750 µL of washing solution was added, and the mixture was centrifuged at 13,000 rpm for 1 minute at room temperature. The eluate was discarded, and the spin column was reinserted into the recovery tube. To remove any remaining washing solution, the spin column was subjected to centrifugation at 13,000 rpm or higher for 1 minute at room temperature. The spin column was then placed into a sterilized microtube, and 15-20 µL of distilled water was added. The mixture was left to stand at room temperature for 1 minute. After 1 minute, the spin column was centrifuged at 13,000 rpm for 1 minute to elute the purified vector. The concentration of the purified vector was then measured using a NanoDrop.

Cloning - Gibson Assembly Test Method (Standard Protocol): A sterilized micro PCR tube was prepared, into which the linear cleaved vector, gene of interest and green fluorescent protein (GFP) were added at a molar concentration ratio of 1:3:3. The final reaction volume was adjusted to 20 µL by adding the following reagents(at that time, the 5× T5 exonuclease-dependent mixed buffer solution was taken from -80°C storage and thawed on ice before use). The Gibson Assembly reaction mixture was prepared as follows.

**[Table 5]**

| Sample | Volume(µl) |
|---|---|
| 5× T5 exonuclease-dependent mixed buffer solution | 4 |
| pcDNA 3.1 vector (100 ng) | 3.66 |
| (8.15 pM (1x), 27.34 ng/ml | |
| Green fluorescent protein (24.45 pM (3x)) | 1 |
| SNAP25 gene | 1 |
| (24.45 pM (3x)) | |
| Distilled water | 10.34 |
| Final volume | 20 |

The order for mixing is as follows: distilled water > green fluorescent protein > gene of interest > the vector > > 5× buffer solution. The mixture was gently mixed using a pipette and centrifuged to collect the solution at the bottom of the tube. The reaction was then carried out in a PCR machine at 37°C for 40 minutes.

Transformation : Approximately 10 minutes before the completion of the Gibson assembly reaction, bacterial cells were taken from storage at -80°C and thawed on ice. Upon completion of the reaction, 2 µL of the vector of the Gibson assembly product was added to 200 µL of thawed bacterial cells. The mixture was gently mixed using a pipette, centrifuged to collect the solution, and incubated on ice for 10 minutes. Following the incubation, the tube was placed in a 42°C heating block for 1 minute and 30 seconds, then immediately returned to ice for an additional 5 minutes. Afterward, 800 µL of antibiotic-free LB medium was added to the cells and incubated at 37°C in a shaking incubator for 1 hour. The cultured cells were then centrifuged at 5,000 rpm for 3 minutes, and 800 µL of the supernatant was discarded. the cell pellet was gently resuspended using the remaining 200 µL of the supernatant. 200 µL of the resuspended culture medium was spread onto an LB agar plate containing ampicillin using a sterile spreader. The plate was inverted and incubated at 37°C for 16 hours. After incubation, colony formation was confirmed, and the plate was sealed and stored at 4°C.

Culture and Purification of Recombinant Vector Gene : Following colony confirmation, 5 µL of 100 mg/mL ampicillin was added to 10 mL of antibiotic-free lysogeny broth (LB) medium(a final concentration of 100 µg/mL) to prepare LB medium containing ampicillin. Subsequently, 5 mL of the antibiotic-supplemented medium was dispensed into a 14 mL round-bottom tube. A single colony from the LB agar plate was picked using a sterile pipette tip and inoculated into the prepared medium. The tube was gently mixed by pipetting, capped loosely, and incubated in a shaking incubator at 37°C for 16 hours. After incubation, the tube was securely sealed and centrifuged at 5,000 rpm for 10 minutes. Plasmid purification was then performed using a plasmid mini prep kit according to the manufacturer's protocol. For each 5 mL culture, 250 µL of pre-chilled resuspension solution (Sol I) (containing RNase A) was added to the pellet, and the pellet was gently resuspended by pipetting. The resuspended cell suspension was transferred to a sterile microcentrifuge tube. Next, 250 µL of lysis solution (Sol II) was added to the tube, and the mixture was gently inverted 5-6 times to lyse the cells and the solution was centrifuged to pellet. Then, 350 µL of neutralization solution (Sol III) was added to the tube, followed by gentle inversion 5-6 times to mix thoroughly. The tube was centrifuged at 13,000 rpm for 10 minutes to pellet the cell debris. The resulting supernatant was carefully transferred to a spin column and centrifuged at 13,000 rpm for 1 minute. The eluate collected in a recovery tube was discarded, and 750 µL of wash buffer was added to the spin column, followed by centrifugation at 13,000 rpm for 1 minute. The eluate collected in a recovery tube was discarded again, and to remove residual wash buffer, the column was centrifuged for an additional 1 minute at 13,000 rpm. The spin column was then transferred to a sterile microcentrifuge tube, and the lid of the column was left open for 30 seconds to allow residual ethanol to evaporate. Subsequently, 25-30 µL of sterile distilled water was added to the center of the spin column and left to stand at room temperature for 1 minute. The tube was then centrifuged at >13,000 rpm for 1 minute to elute the purified plasmid DNA. The concentration of the recovered plasmid was measured using a NanoDrop spectrophotometer. A portion of the purified plasmid was transferred to a sterile microtube and subjected to DNA sequencing analysis to confirm sequence information.

Preparation for establishing transformed cell line is as follows.

Preparation of cell culture medium : 50 mL of 10% fetal bovine serum (FBS) and 5 mL of 1% penicillin antibiotic were added to 500 mL (MEM and DMEM) of cell culture medium in a biosafety cabinet. The mixture was thoroughly mixed, closed with a lid, sealed with parafilm and stored at 4°C (The prepared cluture medium was used within one month and warmed to 37°C in a water bath prior to use).

Preparation of cell culture medium used in transformation : 25 mL of 5% FBS was added to 500 mL of Opti-MEM in a biosafety cabinet. The mixture was mixed well, and the lid was sealed with parafilm, and stored at 4°C (the culture medium was used within one month warmed the culture medium at 37°C water bath prior to use).

Preparation of an extracellular matrix protein(fibronectin) coated plate : a 1 mg/mL of extracellular matrix protein solution was diluted 1:50 with triple-distilled water. 800 µL of the diluted solution was added to each well of a 6-well plate and left to stand at room temperature for 1 hour. Afterward, the extracellular matrix protein solution was discarded, and each well was washed twice with 4 mL of phosphate-buffered saline (PBS). The plates were drained on a sterile towel and air-dried at room temperature for at least 45 minutes.

Preparation of cell culture medium for selecting transformed cell line : 50 mL of 10% FBS and 5 mL of 1% penicillin antibiotic were added to 500 mL of cell culture medium(MEM and DMEM). Subsequently, 5 mL of geneticin(G418) to which the transformed plasmid is resistant, was added and mixed thoroughly. The lid was sealed with parafilm and stored at 4°C (the culture medium was used within one month after warming to 37°C water bath).

Cell thawing and culture : frozen normal cell lines, HEK293, HEK293T, and U2OS were rapidly thawed in a water bath. Each of thawed cell lines was then transferred into a 25 cm² flask with 7 mL of pre-warmed culture medium, and incubated at 37°C in a 5% CO₂ incubator. After confirming cell growth over 1-2 days after thawing, and once cell confluency reached 70-80%, the culture medium in the flask on which the cells were attached was removed by pipetting. 5 mL of PBS was poured into the flask and the culture medium in the flask were gently washed with the PBS, and the washed solution was removed by pipetting. Then, 1 mL of trypsin-EDTA solution was added to the flask, followed by incubation in a 5% CO₂ incubator at 37°C for 3-5 minutes. After incubation, the flask was gently tapped to detach the cells, and detachment was confirmed under a microscope. Next, 5 mL of the prepared culture medium was added, and the culture medium containing the cells was transferred to a 15 mL conical tube. 12 mL of culture medium was added to a 75 cm² flask and 2 mL of the culture medium containing the cells was added to the 75 cm² flask. The flask was closed with a lid and gently distributed the culture medium evenly on bottom of the flask, and incubated at 37°C in a 5% CO₂ incubator for 2-3 days (Depending on the cell growth rate, the ratio was adjusted between 1:2 and 1:5, and it was recommended that cells be passaged twice over at least 7 days after thawing before being used in experiments).

### EXAMPLE 2. Preparation of Heterogeneous transformed cell line

Extracellular matrix protein was coated onto 6-well plates for culturing transformed cells, and the plates were dried for later use.

Preparation of gene sample for transformation (per 1 well plate) : a gene sample and pre-Opti MEM were mixed in a sterile microtube 1 and reacted at room temperature for 5 minutes. Separately, in a sterile microtube 2, Lipofectamine 3000 and pre-Opti MEM were mixed and reacted at room temperature for 5 minutes. After the reaction was completed in the microtube 2, 125 µL of the mixture from microtube 2 and 125 µL from microtube 1 were combined in a sterile microtube. The mixture was gently tapped by a finger, centrifuged to settle the solution, and left to react at room temperature for 20 minutes. During the reaction, when normal cells incubated in a 75 cm² flask reached to 80-90% in cell confluency, the culture medium from the surface to which the cells were attached was gently washed with 5 mL of phosphate-buffered saline (PBS), and removed by pipetting. Subsequently, 1 mL of protease solution was added, and the flask was placed in a 37°C, 5% CO₂ incubator for 3-5 minutes. The flask was taken from the incubator and gently tapped. Then, after confirming cells were detached under a microscope, 5 mL of Opti MEM prepared for transformation medium was added, and the cell culture medium containing the cells was transferred to a 15 mL conical tube. After thoroughly mixing 6 mL of Opti MEM containing cells suspension with a pipet, 100 µL of the mixture was transferred into a sterile microtube. 100 µL of trypan blue solution was added to the microtube containing the cells at ratio of 1:1 and mixed thoroughly, and 10 µL of the mixture was loaded onto a chamber between hemocytometer and glass cover and calculated the number of cells under a microscope. After confirming the number of cells, the mixture was prepared to seed 1.0 × 10⁶ cells per 1 well plate. After the reaction of sample mixture for transformation, 250 µL of the sample was added to a well plate, followed by 1,750 µL of 5% Opti MEM containing 1.0 × 10⁶ cells was added and thereby the final volume was 2 mL per well. The solution was spread on the plate and cultured in a 37°C, 5% CO₂ incubator for 24-48 hours (the transformation time depended on cell type). After 16 hours of transformation, the medium containing the sample mixture for transformation was carefully removed by pipet from each well. To select cells containing only the vector, 2 mL of medium for selecting transformed cell line containing 0.1 mg/mL geneticin antibiotic was gently added to each well. The plate was then cultured in a 37°C, 5% CO₂ incubator for 24-48 hours (the transformation time depended on cell type). After the incubation, the medium of 6 well plates was removed to select transformed cells and eliminate dead cells via pipetting, and 2 mL of fresh medium for selecting transformed cells was gently added to each well. The plates were cultured in a 37°C, 5% CO₂ incubator for 2-4 days (the transformation time depended on cell type). When the cell confluency in the well plates exceeded 90%, the medium was removed through pipetting and 1 mL of PBS was gently added. The cell culture medium from the surface to which the cells were attached was washed by PBS. Then, 1 mL of protease solution was added, and the plates were placed in a 37°C, 5% CO₂ incubator for 3-5 minutes. The plates were taken, tapped gently, and cell detachment was confirmed under a microscope. Next, 3 mL of the medium for selecting transformed cells was added to each well, and the cells in the well plates were diluted by pipetting. 12 mL of the medium was added to a 75 cm² flask and 3mL in the well plates containing cells was added to the flask. The flask was closed with a lid and the medium was spread on the bottom and cultured in a 37°C, 5% CO₂ incubator for 2-3 days (the ratios were adjusted between 1:2 and 1:5 depending on the cell growth rate). The fluorescence expression of the cell line to which fluorescent gene or recombinant fluorescent gene was inserted was confirmed using an image analysis device (see FIG. 2).

### EXAMPLE 3. Botulinum toxin activity assay according to fetal bovine serum concentration in heterogeneous transformed cell line

In a biosafety cabinet, cell culture medium (MEM, DMEM) were prepared at different fetal bovine serum (FBS) concentrations as shown in Table 6, with a final volume of 50 mL.

**[Table 6]**

| Fetal bovine serum (FBS) concentrations (%) | Volume of serum (mL) | Cell culture medium (mL) (no antibiotics) |
|---|---|---|
| 20 | 10 | 50 |
| 15 | 7.5 | 50 |
| 10 | 5 | 50 |
| 5 | 2.5 | 50 |
| 1 | 1 | 50 |
| 0 | 0 | 50 |

When the confluency of the prepared heterogeneous transformed cell lines cultured in two 75 cm² flasks reached 80-90%, 5 mL of phosphate-buffered saline (PBS) was slowly added to each flask to wash the cell culture medium from the surface to which the cells were attached, and then removed by pipetting. Subsequently, 1 mL of protease solution was added, and the flasks were placed in a 37°C, 5% CO₂ incubator for 3-5 minutes. After incubation, the flasks were gently tapped on the side, and detachment of the cells was confirmed under a microscope. Then, 5 mL of cell culture medium was added to each flask, and the cell culture medium was transferred to a 50 mL conical tube. The tubes were centrifuged at 1500 rpm for 3 minutes. After centrifugation, the supernatant was carefully removed from each 15 mL conical tube, and the cell pellets were gently loosened by tapping with a finger. Next, 5 mL of 10% fetal bovine serum (FBS) cell culture medium was added to each tube, and the pellets were thoroughly resuspended. A 5 mL aliquot of each cell culture medium containing cells according to FBS concentration was well mixed by pipetting, and 100 µL of each was transferred into a sterile microtube. To each tube, 100 µL of trypan blue solution was added at a 1:1 ratio and mixed well. Then, 10 µL of the mixture was loaded into a chamber between hemocytometer and the glass cover and the number of the cell was calculated under a microscope. After confirming the number of the cells in the culture medium for selecting transformed cell lines, cell culture medium according to FBS concentration was diluted at 20%, 15%, 10%, 5%, or 1%, and dispensed at 1 mL per concentration so that 8 × 10³ cells were attached per well in a total volume of 90 µL in a 96-well round-bottom plate. In addition, serially diluted botulinum toxin samples to be treated with the cells were prepared as shown in Table 7 below, with a final volume of 300 µL for each.

**[Table 7]**

| No. | Concentrati on (µg/mL) | Original solution to be diluted | botulinum toxin A (µL) | cell culture medium according to FBS concentration (µL) | Final volume (µL) |
|---|---|---|---|---|---|
| 1 | 1000 | - | - | - | - |
| 2 | 1 | No. 1 solution | 0.3 | 299.7 | 300 |
| 3 | 0.33 | No. 2 solution | 100 | 200 | 300 |
| 4 | 0.11 | No. 3 solution | 100 | 200 | 300 |
| 5 | 0.037 | No. 4 solution | 100 | 200 | 300 |
| 6 | 0.012 | No. 5 solution | | | 300 |
| 7 | 0 | - | 0 | 300 | 300 |

Each diluted botulinum toxin sample prepared in the 96-well plates was dispensed at 10 µL per well into two wells for each concentration. Subsequently, 90 µL of the prepared cell culture medium containing cells according to FBS concentration was added to each corresponding well. The plates were then incubated in a 37°C, 5% CO₂ incubator for 48 hours. During the incubation period, the fluorescence expression of the recombinant gene cell lines in response to botulinum toxin treatment and the effects of the different FBS concentrations on fluorescence intensity were assessed using a fluorescence microscope (see FIG. 3 and FIG. 4).

### EXAMPLE 4. Expression level analysis for evaluating the efficacy of botulinum toxin in heterologous transformed cell lines

When the confluency of the prepared heterologous transformed cell lines cultured in two 75 cm² flasks reached 80-90% in a biosafety cabinet, 5 mL of phosphate-buffered saline (PBS) was added to each flask and the cell culture medium from the surface to which the cells were attached was washed by PBS. 1 mL of protease solution was added, and the flasks were incubated in a 37°C, 5% CO₂ incubator for 3-5 minutes. After incubation, the sides of the flasks were gently tapped, and it was confirmed under a microscope that all the cells had detached. 5 mL of the culture medium was added to each flask, and the culture medium containing the cells was transferred into a 50 mL conical tube using a pipette. The solution was centrifuged at 1,500 rpm for 3 minutes. The supernatant was carefully removed from the centrifuged 15 mL conical tube with a pipette, and the cell pellet was gently loosened by tapping. 5 mL of 10% fetal bovine serum (FBS) cell culture medium was added to resuspend the pellet.

After thoroughly mixing 5 mL of the culture medium containing cells, 100 µL of the medium was transferred into a sterile microtube. 100 µL of trypan blue stain was added to the microtube containing the cells at a 1:1 ratio, mixed well, and 10 µL of the mixture was loaded into the chamber between a hemocytometer and a glass cover. The number of cells was then calculated under a microscope.

After determining the number of cells in the culture medium containing cells, the medium was diluted to a volume of 10 mL with cell culture medium so that 1.8 × 10⁴ cells could attach in 100 µL (one well) of a 96-well plate. Serial concentrations of botulinum toxin samples were prepared as shown in Table 7, and the final volume was adjusted to 300 µL.

10 µL of each diluted botulinum toxin sample was dispensed into two wells for each concentration in a 96-well plate, and 90 µL of the cell culture medium according to FBS concentration was added. The medium was incubated for 48 hours in a 37°C, 5% CO₂ incubator. After incubation, expression levels were assessed under a fluorescence microscope to evaluate the efficacy of the botulinum toxin, and the expression levels were analyzed according to the analysis process (FIG. 5 and FIG. 6).

While the specific aspects of the present invention have been described in detail above, it will be apparent to those skilled in the art that such specific descriptions are merely exemplary embodiments and should not be construed as limiting the scope of the invention. Accordingly, the true scope of the present invention is to be defined by the appended claims and their equivalents.

## Claims

1. A recombinant cell for detecting or determining botulinum toxin activity, comprising a gene construct comprising a recombinant fluorescent protein coding sequence, a botulinum toxin recognition sequence and a peptide coding sequence for inhibiting fluorescence expression.

2. The recombinant cell of Claim 1, wherein the cell is a prokaryotic cell, an animal cell or a plant cell.

3. The recombinant cell of Claim 1, wherein the recombinant fluorescent protein is selected from the group consisting of green fluorescent protein (GFP), blue fluorescent protein (BFP), cyan fluorescent protein (CFP), yellow fluorescent protein (YFP), red fluorescent protein (RFP) and orange fluorescent protein (OFP), or is recombinantly produced therefrom.

4. The recombinant cell of Claim 1, wherein the botulinum toxin recognition sequence is a sequence encoding a peptide that serves as a substrate for botulinum toxin and is cleaved by the botulinum toxin.

5. The recombinant cell of Claim 4, wherein the botulinum toxin recognition sequence comprises the 197th amino acid of the amino acid sequence represented by SEQ ID NO. 1 and 6 to 15 adjacent amino acids thereof.

6. The recombinant cell of Claim 1, wherein the peptide is transmembrane domain (TMD) of influenza M2.

7. The recombinant cell of Claim 6, wherein the peptide is produced from a nucleic acid sequence represented by SEQ ID NO. 10.

8. The recombinant cell of Claim 1, wherein the gene construct is introduced using a cell-penetrating peptide, a lipid-based gene carrier, a combination thereof, electroporation or magnetofection.

9. The recombinant cell of Claim 1, wherein the botulinum toxin is botulinum toxin serotype A.

10. A method for detecting or determining botulinum toxin activity using a cell-based assay, comprising:
(a) culturing the recombinant cell according to Claim 1;
(b) treating the cultured recombinant cell with botulinum toxin; and
(c) measuring fluorescence intensity or the number of cells expressing fluorescently in the recombinant cells treated with the botulinum toxin.

11. A method for evaluating a botulinum toxin inhibitor using a cell-based assay, comprising:
(a) culturing recombinant cells of any one of Claim 1-9;
(b) treating the cultured recombinant cells with botulinum toxin and a candidate inhibitor; and determining the activity of the botulinum toxin by measuring fluorescence intensity or the number of cells exhibiting fluorescence and comparing the results with a control group.

12. A kit for detecting or determining botulinum toxin activity, comprising a probe that includes a fluorescent substance, a botulinum toxin-recognition peptide, and a fluorescence expression inhibitor.
